# EUROPEAN PATENT SPECIFICATION

(11) **EP 4 267 153 B1**
(45) Date of publication and mention of the grant of the patent: **14.10.2026**
(21) Application number: 21844974.2
(22) Date of filing: 22.12.2021
(51) Int. Cl.: A61K 35/74, A61K 35/741, C12N 1/20, A61P 29/00, A61P 1/00

(54) **COMPOSITIONS COMPRISING AT LEAST ONE STRAIN OF STAPHYLOCOCCUS CARNOSUS FOR USE IN TREATING OR PREVENTING INFLAMMATION OR MICROBIAL DYSBIOSIS**
ZUSAMMENSETZUNGEN, DIE MINDESTENS EINEN STAMM VON STAPHYLOCOCCUS CARNOSUS ENTHALTEN, ZUR VERWENDUNG BEI DER BEHANDLUNG ODER VORBEUGUNG VON ENTZÜNDUNGEN ODER MIKROBIELLER DYSBIOSE
COMPOSITIONS CONTENANT AU MOINS UNE SOUCHE DE STAPHYLOCOCCUS CARNOSUS ET LEUR UTILISATION DANS LE TRAITEMENT OU LA PRÉVENTION DE L'INFLAMMATION OU DE LA DYSBIOSE MICROBIENNE

(30) Priority: 23.12.2020 US 202063129808 P
(43) Date of publication of application: 01.11.2023
(73) Proprietor: Société des Produits Nestlé S.A., 1800 Vevey (CH)
(72) Inventor: GIANNASCA, Paul, Joseph, Rumney, NH 03266 (US); DUBOUX, Stéphane, 1162 St-Prex (CH)
(74) Representative: Elkington and Fife LLP
(86) International application number: PCT/EP2021/087283
(87) International publication number: WO 2022/136544

(56) References cited:
- MÜLLER ANNE ET AL: "Selection ofStaphylococcus carnosusstrains based on in vitro analysis of technologically relevant physiological activities", ANALS OF MICROBIOLOGY, DISTAM, MILAN, IT, vol. 66, no. 1, 31 July 2015 (2015-07-31), pages 479 - 487, XP035795282, ISSN: 1590-4261, [retrieved on 20150731], DOI: 10.1007/S13213-015-1133-Y
- ROAGER HENRIK M. ET AL: "Microbial tryptophan catabolites in health and disease", NATURE COMMUNICATIONS, vol. 9, no. 1, 1 December 2018 (2018-12-01), XP055882342, Retrieved from the Internet <URL:https://www.nature.com/articles/s41467-018-05470-4.pdf> DOI: 10.1038/s41467-018-05470-4
- JIN-HYUNG LEE ET AL: "Indole as an intercellular signal in microbial communities", FEMS MICROBIOLOGY REVIEWS, vol. 34, no. 4, 1 July 2010 (2010-07-01), pages 426 - 444, XP055420784, DOI: 10.1111/j.1574-6976.2009.00204.x
- KONOPELSKI PIOTR ET AL: "Indoles - Gut Bacteria Metabolites of Tryptophan with Pharmacotherapeutic Potential", CURRENT DRUG METABOLISM, vol. 19, no. 10, 14 September 2018 (2018-09-14), US, pages 883 - 890, XP055913177, ISSN: 1389-2002, DOI: 10.2174/1389200219666180427164731
- KRISHNAN SMITHA ET AL: "Gut Microbiota-Derived Tryptophan Metabolites Modulate Inflammatory Response in Hepatocytes and Macrophages", CELL REPORTS, vol. 23, no. 4, 1 April 2018 (2018-04-01), US, pages 1099 - 1111, XP055913178, ISSN: 2211-1247, DOI: 10.1016/j.celrep.2018.03.109
- ANONYMOUS: "Staphylococcus carnosus carnosus TM300 tryptophan synthase subunit alpha", 27 February 2018 (2018-02-27), XP055913179, Retrieved from the Internet <URL:https://biocyc.org/gene?orgid=SCAR396513&id=SCA_RS05105-MONOMER> [retrieved on 20220418]
- SCHLEIFER K. H. ET AL: "Description of a New Species of the Genus Staphylococcus: Staphylococcus carnosus", INTERNATIONAL JOURNAL OF SYSTEMATIC BACTERIOLOGY, vol. 32, no. 2, 1 April 1982 (1982-04-01), pages 153 - 156, XP055913144, ISSN: 0020-7713, Retrieved from the Internet <URL:https://www.microbiologyresearch.org/docserver/fulltext/ijsem/32/2/ijs-32-2-153.pdf?expires=1650050165&id=id&accname=guest&checksum=685C6AAB697FA41F2500293E73952616> DOI: 10.1099/00207713-32-2-153

## Description

### BACKGROUND

The present disclosure generally relates to compositions comprising at least one *Staphylococcus carnosus* strain, wherein the at least one strain of *S. carnosus* is *S. carnosus* CNCM I-5398 (NCC 971) or *S. carnosus* CNCM I-5400 (NCC 1052), and further relates to methods of making such compositions and methods of using such compositions therapeutically or prophylactically for treating, preventing, reducing an incidence of, and/or reducing a severity of inflammation in an individual; preventing, treating or regulating microbiota dysbiosis in an individual at risk of or suffering from the microbiota dysbiosis; promoting intestinal mucosa healing; or preventing or treating a disorder associated with microbiota dysbiosis in an individual at risk of or having the disorder.

Chronic inflammation is at the core of many human diseases, including inflammatory bowel disease (IBD), liver disease and related metabolic diseases, multiple sclerosis and others. Most anti-inflammatory therapies in use today have deleterious side-effects that limit their long-term utility, such as corticosteroids and antibodies targeting specific immunological pathways.

Further in this regard, IBD is a group of inflammatory conditions of the colon and small intestine. The disease may cause severe abdominal pain and nutritional problems (food intolerance and deficiencies). The major types of IBD are Crohn's disease (CD) and ulcerative colitis (UC). CD and UC mainly differ by their location and nature of the inflammatory changes. CD can affect any part of the gastrointestinal tract, from the oral cavity to the anus, with more common clinical manifestations occurring in the ileum and large intestine. UC is restricted to the colon and the rectum.

The etiology of IBD is still not completely understood. IBD is characterized not only by the mucosal inflammation but also by the severe damage of the intestinal barrier function. Recent clinical studies have featured "mucosal healing" as the most significant prognostic factor for long-term remission in IBD patients and low risk of surgical treatment in CD patients.

Clinical mucosal healing has been defined as complete repair of the epithelial layer and underlying tissue, at both endoscopic and microscopic level. Mucosal healing decreases the relapse risk in patients with inflammatory bowel disease, but the role of dietary supplementation in this process has been inadequately investigated.

### SUMMARY

For preliminary context of the present disclosures, it should be noted tryptophan is an essential amino acid for humans and supplied by dietary protein. Most of the amino acid is absorbed in the small intestine and metabolized via kynurenine and 5-hydroxyindole pathways and unabsorbed tryptophan are catabolized by commensal bacteria in the small intestine and in the colon. Examples of catabolites are indole, tryptamine, indole-ethanol, indole-propionic acid, indole-lactic acid (ILA), indole-acetic acid (IAA), skatole, indole-aldehyde (IAld) and indole-acrylic acids.

These metabolites may affect mucosal homeostasis, appetite control, gastrointestinal mobility and/or immune responses through gut epithelial cells. In particular, several tryptophan metabolites such as IAA, IAld, IA, ILA, tryptamine, and skatole act on the aryl hydrocarbon receptor (AHR) found in intestinal immune cells and consequently modulate immune responses in AHR dependent manner. Additionally, it acts through G-protein receptors and signaling pathways, including nuclear factor erythroid 2-related factor 2 (Nrf2), to regulate oxidative stress and inflammatory responses.

Due to those mucosal homeostatic and immunomodulatory effects of tryptophan metabolites or indole derivatives, the present inventors believe, without being bound by theory, that *S. carnosus* can potentially produce these metabolites through catabolic enzymes and thus improve gut health in IBD patients or in the general population. As an essential amino acid, ingestion of tryptophan influences physiological levels of tryptophan in the body (i.e., blood, brain and gut), consequently stimulating serotonin/melatonin synthesis through aforementioned pathways. Initial levels of tryptophan in diet can affect not only bioavailability of tryptophan but also availability of tryptophan metabolites in the gut. Hence, an aspect of the present disclosure is based on modulating (e.g., increasing) the tryptophan metabolites generated by gut microbiome.

Further in this regard, food-grade and/or food-derived bacterial strains have a long history of human exposure with a good safety profile, and some of these strains have been developed as probiotics for ameliorating certain ailments, such as gastrointestinal dysfunction, e.g., constipation, diarrhea, pain and/or bloating. The mechanism(s) by which probiotics provide relief are not well understood, and thus the selection of specific strains has been a largely empirical exercise. Nevertheless, probiotics are generally regarded as safe, have a history of safe consumption, and are used in food manufacturing.

More recently, as the interplay of the gut microbiome and the host is becoming better understood, it is becoming clear that the benefits bacteria provide to the host are often mediated by the specific molecules that they produce. With this in mind, the inventors examined host physiological pathways that bacterial metabolites can engage and selected the aryl hydrocarbon receptor (AHR) pathway as a target.

The AHR acts as an environmental sensor by recognizing a family of molecules called indole derivatives, which include the metabolites produced by the breakdown of tryptophan. Both the host and certain bacteria can catabolize tryptophan into related molecules that engage the AHR and stimulate many downstream effector functions, which in the gut includes proliferation of regulatory T cells (Tregs) and epithelial cells, among other activities. The net effects of proliferation of Tregs and epithelial cells are dampening of inflammation and promoting mucosal healing/restoring mucosal intestinal function. IBD patients have aberrant complement of gut bacterial taxa (dysbiosis) and, consequently, their gut microbiota have a reduced capacity to catabolize tryptophan and activate the AHR. Some other inflammatory diseases are likely associated with a reduced capacity of the gut microbiota to catabolize tryptophan. Furthermore, antibiotic treatment is known to adversely affect the healthy balance of gut bacteria which can lead to impaired physiological and physical (e.g. barrier) functions and reduced defense against pathogenic organisms.

The present disclosure addresses the reduced ability of an altered gut microbiota to generate beneficial metabolites and maintain protective functions by supplementing the host with safe, food-grade and/or food-derived bacteria that have a high capacity to process tryptophan into bioactive metabolites the engage the AHR and activate multiple health-promoting functions.

Most interventions for addressing chronic inflammation have adverse effects on the host, but food-grade bacteria have a long history of safe human consumption and are amenable to long-term use. Hence, the use of beneficial bacteria to replenish important physiological capabilities associated with a healthy gut microbiota in a dysbiotic host may be a promising approach.

Additional features and advantages are described herein and will be apparent from the following Figures and Detailed Description.

### BRIEF DESCRIPTION OF DRAWINGS

**FIG. 1****.**

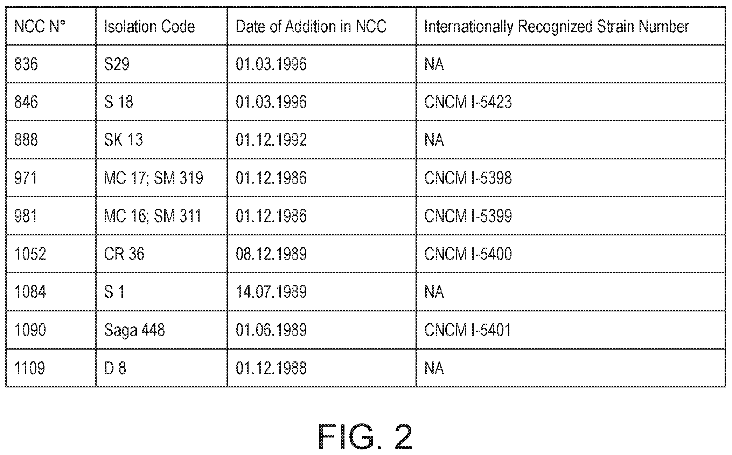

**FIG. 2** is a table of selected NCC *S. carnosus* strains in the first experimental example disclosed herein.
**FIG. 3** depicts the evaluation of *S. carnosus* in a mouse DSS colitis model in a second experimental example disclosed herein.
**FIG. 4** shows a heat map of changes in cytokine levels in colonic tissue harvested at study terminus (Day 14), with study group values depicted as relative to the DSS vehicle control (shown as "0") .
**FIG. 5** is a chart showing disease activity index (DAI) at peak of disease (Day 10), statistical comparisons to vehicle control, in the second experimental example disclosed herein.
**FIGS. 6A** **and** **6B** present charts showing bacterial-derived tryptophan metabolites activate AHR in a dose-dependent manner in a third experimental example disclosed herein.
**FIGS. 7A** and **7B** present charts showing bacterial-derived tryptophan metabolites demonstrate low level activation of AHR and with limited potentiation compared to TCDD in the third experimental example disclosed herein.
**FIG. 8** is a chart of results from an initial test of metabolites in culture supernatants in the third experimental example disclosed herein, all analytes quantified from a single sample.
**FIG. 9** shows NST-04 Mouse Colon; Mean Sum Colitis Scores. Group mean +/standard error of the mean (SEM). For mice administered DSS, distal sum colitis scores were reduced in mice treated with NCC971 or anti-p40 in comparison to the vehicle control treatment. These same reductions were not observed in the proximal colon. Comparative reductions were minimal or absent in mice treated with NCC1052 or the combination of NCC971 + NCC1052 in the proximal segment and distal segment of colon.
**FIG. 10** shows NST-04 Mouse Proximal Segment of Colon; Mean Histopathology Scores. Group mean +/- SEM. Amongst-group trends were similar to those observed in the proximal segment sum colitis scores
**FIG. 11** shows Mouse Distal Segment of Colon; Mean Histopathology Scores. Group mean +/- SEM. Amongst-group trends were similar to those observed in the distal segment sum colitis scores
**FIG. 12** shows NST-04 Mouse Proximal Segment of Colon; Mean Goblet Cell Abundance Scores. Group mean +/- SEM. Goblet cell abundance was primarily increased. Slight reductions in the decreased and increased abundance scores were associated with anti-p40 treatment in comparison the vehicle control, but this did not translate to differences in the sum scores.
**FIG. 13** shows NST-04 Mouse Distal Segment of Colon; Mean Goblet Cell Abundance Scores. Group mean +/- SEM. Goblet cell abundance was primarily decreased. Slight reductions in the decreased and increased abundance scores were associated with NCC971 or anti-p40 treatment in comparison the vehicle control, but this translated to relatively minimal differences in sum scores.
**FIG. 14** shows NST-04 Mouse Colon; Mean Sub-Cryptal Mucosal Thickness Measurements. Group mean +/- SEM. For mice administered DSS, thickness measurements in the distal colon were reduced in mice treated with NCC971 or anti-p40 in comparison to the vehicle control treatment. These same reductions were not observed in the proximal colon. Comparative reductions were not observed in in mice treated with NCC1052 or the combination of NCC971 + NCC1052 in the proximal segment and distal segment of colon.

### DETAILED DESCRIPTION

### Definitions

Some definitions are provided hereafter. Nevertheless, definitions may be located in the "Embodiments" section below, and the above header "Definitions" does not mean that such disclosures in the "Embodiments" section are not definitions.

All percentages expressed herein are by weight of the total weight of the composition unless expressed otherwise. As used herein, "about," "approximately" and "substantially" are understood to refer to numbers in a range of numerals, for example the range of -10% to +10% of the referenced number, preferably -5% to +5% of the referenced number, more preferably -1% to +1% of the referenced number, most preferably -0.1% to +0.1% of the referenced number. All numerical ranges herein should be understood to include all integers, whole or fractions, within the range. Moreover, these numerical ranges should be construed as providing support for a claim directed to any number or subset of numbers in that range. For example, a disclosure of from 1 to 10 should be construed as supporting a range of from 1 to 8, from 3 to 7, from 1 to 9, from 3.6 to 4.6, from 3.5 to 9.9, and so forth.

As used in this disclosure and the appended claims, the singular forms "a," "an" and "the" include plural referents unless the context clearly dictates otherwise. Thus, for example, reference to "a bacterial strain" or "the bacterial strain" means "the bacterial strain" and includes two or more the bacterial strains.

The words "comprise," "comprises" and "comprising" are to be interpreted inclusively rather than exclusively. Likewise, the terms "include," "including" and "or" should all be construed to be inclusive, unless such a construction is clearly prohibited from the context. Nevertheless, the compositions disclosed herein may lack any element that is not specifically disclosed herein. Thus, a disclosure of an embodiment using the term "comprising" includes a disclosure of embodiments "consisting essentially of" and "consisting of" the components identified.

The terms "at least one of" and "and/or" used in the respective context of "at least one of X or Y" and "X and/or Y" should be interpreted as "X," or "Y," or "X and Y." For example, "at least one of *S. carnosus* CNCM I-5398 or *S. carnosus* CNCM I-5400" should be interpreted as *"S. carnosus* CNCM I-5398 without *S. carnosus* CNCM I-5400," *or "S. carnosus* CNCM I-5400 without *S. carnosus* CNCM I-5398," or "both *S. carnosus* CNCM I-5398 and S. *carnosus* CNCM I-5400." These strains in particular may be used alone or in combination with each other, and/or other strains of *S. carnosus.*

The terms "at least one" is to be interpreted to include one or more, for example 1, 2, 3, 4, 5, 6, 7, 8, 9, 10 or more. The terms "at least one *Staphylococcus carnosus* strain" is to be interpreted to include one single *Staphylococcus carnosus* strain alone as well as combinations of 2, 3, 4, 5, 6, 7, 8, 9, 10 or more strains. In the context of "at least one" *Staphylococcus carnosus* strain that produces one or more of tryptamine, indole, indole propionic acid, 3-methylindole, indole-3-acetic acid or indole-3-acetaldehyde, the term is to be interpreted to include one or more strains of *Staphylococcus carnosus,* for example 1, 2, 3, 4, 5, 6, 7, 8, 9, 10 or more strains, wherein each strain produces one or more of tryptamine, indole, indole propionic acid, 3-methylindole, indole-3-acetic acid or indole-3-acetaldehyde. Each strain of *Staphylococcus carnosus* may produce one or more of tryptamine, indole, indole propionic acid, 3-methylindole, indole-3-acetic acid or indole-3-acetaldehyde. Where two or more strains are present in combination, each strain may produce the same or different metabolite and/or metabolites. For example, "at least one *Staphylococcus carnosus* strain that produces one or more of tryptamine, indole, indole propionic acid, 3-methylindole, indole-3-acetic acid or indole-3-acetaldehyde" includes any combination of strains that produce any combination of the listed metabolites, wherein each strain may produce one metabolite alone or more metabolites in any combination.

Where used herein, the terms "example" and "such as," particularly when followed by a listing of terms, are merely exemplary and illustrative and should not be deemed to be exclusive or comprehensive. As used herein, a condition "associated with" or "linked with" another condition means the conditions occur concurrently, preferably means that the conditions are caused by the same underlying condition, and most preferably means that one of the identified conditions is caused by the other identified condition.

"Prevention" includes reduction of risk and/or severity of a condition or disorder. The terms "treatment" and "treat" include both prophylactic or preventive treatment (that prevent and/or slow the development of a targeted pathologic condition or disorder) and curative, therapeutic or disease-modifying treatment, including therapeutic measures that cure, slow down, lessen symptoms of, and/or halt progression of a diagnosed pathologic condition or disorder; and treatment of patients at risk of contracting a disease or suspected to have contracted a disease, as well as patients who are ill or have been diagnosed as suffering from a disease or medical condition. The terms "treatment" and "treat" do not necessarily imply that a subject is treated until total recovery. The terms "treatment" and "treat" also refer to the maintenance and/or promotion of health in an individual not suffering from a disease but who may be susceptible to the development of an unhealthy condition. The terms "treatment" and "treat" are also intended to include the potentiation or otherwise enhancement of one or more primary prophylactic or therapeutic measures. As non-limiting examples, a treatment can be performed by a patient, a caregiver, a doctor, a nurse, or another healthcare professional.

As used herein, a prophylactically or therapeutically "effective amount" is an amount that prevents a deficiency, treats a disease or medical condition in an individual, or, more generally, reduces symptoms, alters expression of biomarkers associated with a disease or condition, manages progression of the disease, or provides a nutritional, physiological, or medical benefit to the individual. The relative terms "promote," "improve," "increase," "enhance" and the like refer to a level of a characteristic of the subject (e.g., intestinal mucosa function, goblet cell function or any other indicator of intestinal health) after administration of the composition disclosed herein, which comprises at least one *S. carnosus* strain, relative to the level of the characteristic immediately prior to the administration.

As used herein, the terms "food," "food product" and "food composition" mean a product or composition that is intended for oral ingestion by a human or other mammal and comprises at least one nutrient for the human or other mammal.

"Nutritional compositions" and "nutritional products," as used herein, include any number of food ingredients and possibly optional additional ingredients based on a functional need in the product and in full compliance with all applicable regulations. The optional ingredients may include, but are not limited to, conventional food additives, for example one or more, acidulants, additional thickeners, buffers or agents for pH adjustment, chelating agents, colorants, emulsifies, excipient, flavor agent, mineral, osmotic agents, a pharmaceutically acceptable carrier, preservatives, stabilizers, sugar, sweeteners, texturizers, and/or vitamins. The optional ingredients can be added in any suitable amount.

"Probiotic" means microbial cell preparations or components of microbial cells with a beneficial effect on the health or well-being of the host. (Salminen S, Ouwehand A. Benno Y. et al "Probiotics: how should they be defined" Trends Food Sci. Technol. 1999: 10 107-10).

The term "unit dosage form," as used herein, refers to physically discrete units suitable as unitary dosages for human and animal subjects, each unit containing a predetermined quantity of the composition disclosed herein in an amount sufficient to produce the desired effect, in association with a pharmaceutically acceptable diluent, carrier or vehicle. The specifications for the unit dosage form depend on the particular compounds employed, the effect to be achieved, and the pharmacodynamics associated with each compound in the host.

A "subject" or "individual" is a mammal, preferably a human.

Strains disclosed in the present application have been deposited in the depositary institution indicated in the table below (Table 1), and have received the following date of deposit and accession number:

**Table 1**

| # | NCC Code | Depositary institution | Accession number | Date of deposit |
|---|---|---|---|---|
| 1 | 846 | CNCM | I-5423 | 16/05/2019 |
| 2 | 971 | CNCM | I-5398 | 01/02/2019 |
| 3 | 981 | CNCM | I-5399 | 01/02/2019 |
| 4 | 1052 | CNCM | I-5400 | 01/02/2019 |
| 5 | 1090 | CNCM | I-5401 | 01/02/2019 |

CNCM refers to Collection nationale de cultures de micro-organismes, Institut Pasteur, 28, rue du Dr Roux, F-75724 Paris Cedex 15, France.

Strains 1-5 have been deposited by Nestec S.A., avenue Nestlé 55, 1800 Vevey, Switzerland. Since then, Nestec S.A. has merged into Société des Produits Nestlé S.A. Accordingly, Société des Produits Nestlé S.A. is the successor in title of Nestec S.A., under article 2(ix) of the Budapest Treaty.

### Embodiments

As detailed in the experimental examples later herein, *in silico* screening of food-grade bacterial library for genes encoding enzymes that catabolize tryptophan identified *Staphylococcus carnosus* strains that possess multiple genes for enzymes that are part of established tryptophan catabolic pathways. Based on the presence of multiple genes, these strains are expected to produce multiple tryptophan catabolites. These breakdown molecules are bio-active molecules that can engage the mammalian aryl hydrocarbon receptor (AHR) to elicit a cascade of downstream effector functions.

Furthermore, targeted metabolomic screening of bacterial culture supernatants confirmed that metabolites predicted by in silico screening are indeed produced in culture. Still further, evaluation of purified metabolites in *in vitro* mouse and human AHR binding and activation assays demonstrated that the metabolites bind the mouse and human receptors and activate the reporter gene. The activation level was found to be higher for the human AHR receptor compared to mouse. Importantly, the level and potentiation of activation was ~2,000X lower than that observed for the dioxin reference molecule, which is toxic to the host.

Moreover, evaluation of various *S. carnosus* strains in a mouse colitis model identified certain strains that can significantly reduce disease manifestations. Still further, evaluation of colonic tissue cytokines from the mouse colitis studies has established that a general dampening of pro-inflammatory cytokines is observed in mice administered the efficacious strains.

The identified *Staphylococcus carnosus* strains possess a higher number of tryptophan catabolic enzymes and likely have a higher capacity to produce these bioactive metabolites relative to other probiotic strains, including *Lactobacillus* strains such as *L. hilgardii, L. farraginis, L. buchneri, L. fermentum, L. reuteri and L. paracasei.*

Accordingly, the present invention is defined in the appended claims. Aspects of the disclosure that are not claimed have been retained for reference purposes only and do not form part of the claimed invention.

The present invention provides at least one *Staphylococcus carnosus* strain for use in
(a) treating, preventing, reducing an incidence of, and/or reducing a severity of inflammation in an individual,
(b) preventing, treating or regulating microbiota dysbiosis in an individual at risk of or suffering from the microbiota dysbiosis;
(c) promoting intestinal mucosa healing; or
(d) preventing or treating a disorder associated with microbiota dysbiosis in an individual at risk of or having the disorder;
wherein the at least one *Staphylococcus carnosus* strain produces one or more of tryptamine, indole, indole propionic acid, 3-methylindole, indole-3-acetic acid or indole-3-acetaldehyde; and wherein the at least one *Staphylococcus carnosus* strain comprises at least one of *S. carnosus* CNCM I-5398 or *S. carnosus* CNCM I-5400

A further aspect of the present disclosure is *S. carnosus* CNCM I-5398 or *S. carnosus* CNCM I-5400 alone or in combination for use in a method of treating, preventing, reducing an incidence of, and/or reducing a severity of inflammation, such as a gut inflammatory disease, for example IBD, by administering a therapeutically effective or prophylactically effective amount of at least one *Staphylococcus carnosus* strain that produces one or more of tryptamine, indole, indole propionic acid, 3-methylindole, indole-3-acetic acid or indole-3-acetaldehyde, for example at least one of the strains of *Staphylococcus carnosus* listed in Table 1, for example at least one of *S. carnosus* CNCM I-5398 or *S. carnosus* CNCM I-5400. The effective amount of at least one *Staphylococcus carnosus* strain can catabolize tryptophan into metabolites that engage the aryl hydrocarbon receptor (AHR) of the individual and thereby stimulate proliferation of regulatory T cells (Tregs) and epithelial cells.

Another aspect of the present disclosure is *S. carnosus* CNCM I-5398 or *S. carnosus* CNCM I-5400 alone or in combination for use in a method of promoting intestinal mucosa healing by administering a therapeutically effective or prophylactically effective amount of at least one of the *Staphylococcus carnosus* strains that produces one or more of tryptamine, indole, indole propionic acid, 3-methylindole, indole-3-acetic acid or indole-3-acetaldehyde. For example at least one of the strains of *Staphylococcus carnosus* listed in Table 1, for example at least one of *S. carnosus* CNCM I-5398 or *S. carnosus* CNCM I-5400. The effective amount of at least one *Staphylococcus carnosus* strain can catabolize tryptophan into metabolites that engage the aryl hydrocarbon receptor (AHR) of the individual and thereby stimulate proliferation of epithelial cells.

Yet another aspect of the present disclosure is *S. carnosus* CNCM I-5398 or *S. carnosus* CNCM I-5400 alone or in combination for use in a method of regulating gut microbiota dysbiosis or treating or preventing a disorder associated with microbiota dysbiosis by administering a therapeutically effective or prophylactically effective amount of at least one of the *Staphylococcus carnosus* strain that produces one or more of tryptamine, indole, indole propionic acid, 3-methylindole, 3-methylindole, indole-3-acetic acid or indole-3-acetaldehyde. For example at least one of the strains of *Staphylococcus carnosus* listed in Table 1, for example at least one of *S. carnosus* CNCM I-5398 *or S. carnosus* CNCM I-5400.

In any and all embodiments of the invention relating to the use of *S. carnosus* CNCM I-5398 or *S. carnosus* CNCM I-5400, the strains of *Staphylococcus carnosus* listed in Table 1 may be used alone or in any combination with other strains of *Staphylococcus carnosus* listed in Table 1 and/or other strains of *Staphylococcus carnosus* that produces one or more of tryptamine, indole, indole propionic acid, 3-methylindole, indole-3-acetic acid or indole-3-acetaldehyde but are not listed in Table 1. For example, in any and all aspects of the invention the at least one strain of *S. carnosus* may be *S. carnosus* CNCM I-5398 or *S. carnosus* CNCM I-5400 alone or in combination.

Herein described but not part of the claimed invention are edible or food compositions comprising a therapeutically effective or prophylactically effective amount of at least one *Staphylococcus carnosus* strain that produces one or more of tryptamine, indole, indole propionic acid, 3-methylindole, 3-methylindole, indole-3-acetic acid or indole-3-acetaldehyde, for example at least one of the strains of *Staphylococcus carnosus* listed in Table 1, for example at least one of *S. carnosus* CNCM I-5398 or *S. carnosus* CNCM I-5400. In a specific aspect, the strain of *S. carnosus* is *S. carnosus* CNCM I-5398 or *S. carnosus* CNCM I-5400 alone or in combination. The edible or food composition can be selected from the group consisting of a nutritionally complete product, a drink, a dietary supplement, a meal replacement, a food additive, a supplement to a food product, a powder for dissolution, an enteral nutrition product, an infant formula, and combinations thereof.

Herein described but not claimed as part of the invention are edible or food compositions comprising a therapeutically effective or prophylactically effective amount of at least one *Staphylococcus carnosus* strain that produces one or more of tryptamine, indole, indole propionic acid, 3-methylindole, 3-methylindole, indole-3-acetic acid or indole-3-acetaldehyde for use in treating inflammation in an individual. The at least one of the strains of *Staphylococcus carnosus* may be strains listed in Table 1, for example at least one of *S. carnosus* CNCM I-5398 or *S. carnosus* CNCM I-5400. In a specific aspect, the strain of *S. carnosus* is *S. carnosus* CNCM I-5398 or *S. carnosus* CNCM I-5400 alone or in combination.

The at least one *Staphylococcus carnosus* strain may be administered to the individual as a daily dose of 1x10³ to 1x10¹², preferably 1x10⁷ to 1x10¹¹ cfu (cfu=colony forming unit). The at least one *Staphylococcus carnosus* strain may be administered in a composition comprising between 1x10³ to 1x10¹² cfu/g of dry composition.

The at least one *Staphylococcus carnosus* strain may be alive, fragmented, or in the form of fermentation products (e.g., supernatant) or metabolites, or a mixture of any or all of these states.

In some embodiments, the at least one *Staphylococcus carnosus* strain is administered in a composition further comprising one or more additional probiotics, and the at least one *Staphylococcus carnosus* is a majority of a total amount of probiotics in the composition. For example, the amount of any Lactobacillus in the composition is preferably less than the amount of the at least one *Staphylococcus carnosus.* In some embodiments, the at least one *Staphylococcus carnosus* is the only probiotic in the composition.

The at least one *Staphylococcus carnosus* strain can be administered to the individual by at least one route selected from the group consisting of oral, topical, enteral and parenteral. For example, the at least one *Staphylococcus carnosus* strain can be administered in a composition selected from the group consisting of a nutritionally complete product, a drink, a dietary supplement, a meal replacement, a food additive, a supplement to a food product, a powder for dissolution, an enteral nutrition product, an infant formula, and combinations thereof.

In some embodiments, the at least one *Staphylococcus carnosus* strain can be administered in an amount effective to treat, prevent, reduce an incidence of, and/or reduce a severity of inflammation. Non-limiting examples of such inflammation can be selected from the group consisting of acute inflammation, skin inflammation, inflammatory bowel disease (IBD) including Crohn's disease and/or ulcerative colitis, irritable bowel syndrome, liver inflammation (NASH,NAFLD, alcohol-induced liver injury), allergy, atopy, bone inflammation, rheumatoid arthritis, systemic lupus, Gougerot-Sjogren's syndrome, Reiter's syndrome, poliomyelitis, dermato-myositis, thyroiditis, Basedow, Hashimoto, type I diabetes, Addison's disease, auto-immunes hepatitis, celiac disease, Biermer's disease, multiple sclerosis, myasthenia, eye inflammation, obesity-associated inflammation, age-related low-grade inflammation, Blau's syndrome, Alzheimer's disease, cardiovascular diseases, atherosclerosis, metabolic syndrome, type II diabetes, gingivitis, paronditis, food sensitivities, Celiac disease, and combinations thereof.

The inflammation treated or prevented by the at least one *Staphylococcus carnosus* strain can be IBD, for example Crohn's disease or ulcerative colitis.

In some embodiments, the individual is selected from the group consisting of an infant, a child, an adolescent, an adult and an elderly person.

Preferably the at least one *Staphylococcus carnosus* strain is administered in a composition further comprising at least one component selected from the group consisting of a prebiotic, an amino acid, a protein, a nucleotide, a vitamin, a fish oil, a non-marine source of omega-3 fatty acids, a phytonutrient, an antioxidant, and mixtures thereof.

"Prebiotic" means a food substance that promote the growth of beneficial bacteria in the intestines. A prebiotic is not broken down in the stomach or absorbed in the GI tract of the individual ingesting the prebiotic, but the prebiotic is fermented by the gastrointestinal microflora and/or by probiotics. The addition of a prebiotic is beneficial because the combination of the prebiotic with the at least one *Staphylococcus carnosus* strain delivers synergistic health effects. A composition comprising a combination of a prebiotic and a probiotic is commonly known as a symbiotic composition.

The prebiotics that may be used with the at least one *Staphylococcus carnosus* strain are not particularly limited and include all food substances that promote the growth of probiotics in the intestine. Preferably, the prebiotic may be selected from the group consisting of oligosaccharides, optionally containing fructose, galactose, mannose; dietary fibers, in particular soluble fibers, soy fibers; inulin; or mixtures thereof. Preferred prebiotics are fructo-oligosaccharides (FOS), galacto-oligosaccharides (GOS), isomalto-oligosaccharides, xylo-oligosaccharides, oligosaccharides of soy, glycosylsucrose (GS), lactosucrose (LS), lactulose (LA), palatinose-oligosaccharides (PAO), malto-oligosaccharides, pectins and/or hydrolysates thereof.

The strain for the use according to the invention may be administered as a composition. The composition may be a food product, an animal food product, or a pharmaceutical composition. For example, the product may be a nutritional composition, a nutraceutical, a drink, a food additive or a medicament.

A food additive or a medicament may be in the form of tablets, capsules, pastilles, a liquid, or a powder in a sachet, for example. Food additives or medicaments are preferably provided as sustained release formulations, allowing a substantially constant supply of the at least one *Staphylococcus carnosus* strain for prolonged times.

The composition may be selected from the group consisting of milk powder based products; instant drinks; ready-to-drink formulations; nutritional powders; nutritional liquids; milk-based products, in particular yoghurts or ice cream; cereal products; beverages; water; coffee; cappuccino; malt drinks; chocolate flavoured drinks; culinary products; soups; tablets; and/or syrups.

The composition may optionally comprise any milk obtainable from animal or plant sources, such as one or more of cow's milk, human milk, sheep milk, goat milk, horse milk, camel milk, rice milk or soy milk. Additionally or alternatively, milk-derived protein fractions or colostrum may be used.

The composition comprising at least one *Staphylococcus carnosus* strain may further contain protective hydrocolloids (such as gums, proteins, modified starches), binders, film forming agents, encapsulating agents/materials, wall/shell materials, matrix compounds, coatings, emulsifiers, surface active agents, solubilizing agents (oils, fats, waxes, lecithins etc.), adsorbents, carriers, fillers, co-compounds, dispersing agents, wetting agents, processing aids (solvents), flowing agents, taste masking agents, weighting agents, jellifying agents, gel forming agents, antioxidants and antimicrobials. The composition comprising at least one *Staphylococcus carnosus* strain may also contain conventional pharmaceutical additives and adjuvants, excipients and diluents, including, but not limited to, water, gelatine of any origin, vegetable gums, ligninsulfonate, talc, sugars, starch, gum arabic, vegetable oils, polyalkylene glycols, flavouring agents, preservatives, stabilizers, emulsifying agents, buffers, lubricants, colorants, wetting agents, fillers, and the like. Further, the composition comprising at least one *Staphylococcus carnosus* strain may contain an organic or inorganic carrier material suitable for oral or enteral administration as well as vitamins, minerals trace elements and other micronutrients in accordance with the recommendations of Government bodies such as the USRDA.

The composition comprising at least one *Staphylococcus carnosus* strain may optionally contain a protein source, a carbohydrate source and/or a lipid source, particularly in embodiments of the composition that are a food product.

Any suitable dietary protein may be used, for example animal proteins (such as milk proteins, meat proteins and egg proteins); vegetable proteins (such as soy protein, wheat protein, rice protein, and pea protein); mixtures of free amino acids; or combinations thereof. Milk proteins such as casein and whey, and soy proteins are particularly preferred.

If the composition includes a fat source, the fat source more preferably provides 5% to 40% of the energy of the formula; for example 20% to 30% of the energy. DHA may be added. A suitable fat profile may be obtained using a blend of canola oil, corn oil and high-oleic acid sunflower oil.

A source of carbohydrates may preferably provide between 40% to 80% of the energy of the composition. Any suitable carbohydrate may be used, for example sucrose, lactose, glucose, fructose, corn syrup solids, maltodextrins, and mixtures thereof.

The composition comprising at least one *Staphylococcus carnosus* strain may be administered to humans or animals, in particular companion animals, pets or livestock. It has beneficial effects for any age group. Preferably, the composition is formulated for administration to infants, juveniles, adults or elderly. In some embodiments, the composition be administered to mothers during pregnancy and lactation to treat the infant.

In an embodiment, the composition comprising at least one *Staphylococcus carnosus* strain can be administered for at least 10, 20, 24, 30, 40, 42, 50, or 60 weeks. The composition can preferably be administered between 10 and 60 weeks, between 20 and 50 weeks, between 15 and 30 weeks, or between 35 and 45 weeks.

The composition comprising at least one *Staphylococcus carnosus* strain can maintain or improve the mucosa health status of an IBD patient, as indicated by the maintenance of an endoscopic-proven healthy mucosa.

The maintenance of the endoscopic-proven healthy mucosa can be assessed by the simple endoscopic score-Crohn's Disease (SES-CD). Thus, the improvement of the endoscopic-proven healthy mucosa can be indicated by a reduction in the mean SES-CD score from the onset of administration of the composition comprising at least one *Staphylococcus carnosus* strain to a time point between 20 and 45 weeks, preferably 20 and 30 weeks, for example week 24, after the onset of administration of the composition comprising at least one *Staphylococcus carnosus* strain (endoscopic improvement).

The maintenance of the endoscopic-proven healthy mucosa can be indicated by a maintenance of the endoscopic response, wherein an endoscopic response is indicated as effecting an SES-CD decrease of at least 3 points from the onset of administration of the composition comprising at least one *Staphylococcus carnosus* strain to a time point between 20 and 45 weeks, preferably between 20 and 30 weeks, for example week 24, after the onset of administration of the composition comprising at least one *Staphylococcus carnosus* strain.

The maintenance of the endoscopic-proven healthy mucosa can also be indicated by a maintenance of the clinical remission, wherein clinical remission is indicated as effecting a CDAI of less than 150 points at a time point of between 20 and 45 weeks, preferably 20 and 30 weeks, for example week 24, after the onset of administration of the composition comprising at least one *Staphylococcus carnosus* strain.

The composition comprising at least one *Staphylococcus carnosus* strain can prolong the time until endoscopic or clinical relapse. The composition can thereby reduce the economic impact of CD as indicated by surgery, hospitalization and CD complication rates.

The composition comprising at least one *Staphylococcus carnosus* strain can improve quality of life as indicated, for example, by IBDQ, SF-36v2 and EQ-5 determined at a time point of between 20 and 45 weeks, preferably 20 and 30 weeks, for example week 24, after the onset of administration of the composition comprising at least one *Staphylococcus carnosus* strain.

The composition comprising at least one *Staphylococcus carnosus* strain can improve the composition and functionality of the gut microbiome.

The composition comprising at least one *Staphylococcus carnosus* strain can improve non-invasive biomarkers like CRP or fecal calprotectin.

The composition comprising at least one *Staphylococcus carnosus* strain can be combined with standard treatments common in the treatment of IBD. These standard treatments comprise surgery, antibiotics, immuno-suppressants and anti-inflammatory drugs. Immuno-suppressants can be selected from the group consisting of prednisone, TNF or TNFalpha inhibitors (e.g. infliximab, adalimumab), azathioprine (Imuran), methotrexate, and 6-mercaptopurine. A preferred anti-inflammatory drug is mesalamine (USAN) or 5-aminosalicylic acid (5-amino-2-hydroxybenzoic acid, 5-ASA).

Preferably, the composition comprising at least one *Staphylococcus carnosus* strain is administered in combination with at least one TNF inhibitor or TNF inhibitor therapy. Preferably, the at least one TNF inhibitor is a TNF alpha inhibitor. Preferably the at least one TNF alpha inhibitor is infliximab or adalimumab and most preferred the TNF alpha inhibitors are a combination of infliximab and adalimumab.

Preferred is the administration of any of the above cited immuno-suppressants and anti-inflammatory drugs in combination with the composition comprising at least one *Staphylococcus carnosus* strain. The combination results in a cooperative effect of the administered compounds.

The composition comprising at least one *Staphylococcus carnosus* strain can be used in the treatment of IBD, the treatment of a subject in remission of IBD, or preventing or delaying a relapse of IBD in a subject, wherein the composition comprising at least one *Staphylococcus carnosus* strain is administered in combination with a medicament effective against IBD. The medicament preferably is an immunosuppressant or 5-aminosalicylic acid (5-ASA). This subject could be a subject that has undergone surgery or will undergo surgery. The administration of the composition comprising at least one *Staphylococcus carnosus* strain can occur before, during or after the administration of the medicament.

In some embodiments, the composition comprising at least one *Staphylococcus carnosus* strain for use according to the invention can promote intestinal mucosal healing. In such embodiments, the composition comprising at least one *Staphylococcus carnosus* strain can be administered to an individual who has damaged intestinal mucosa.

In some embodiments, the composition comprising at least one *Staphylococcus carnosus* strain for use according to the invention can regulate gut microbiota dysbiosis or treat or prevent a disorder associated with microbiota dysbiosis. Microbiota dysbiosis is a significant deviation from a balanced microbiota, in terms of global microbiota profile, metabolism or levels of particular taxa. Microbiota dysbiosis is usually associated with and increased vulnerability to disease. For example, reduced levels of Bifidobacterium are associated with increased risk of infection and other pathologies in infants.

Microbiota dysbiosis may be induced by C-section delivery, premature birth, exposure to antibiotics in utero, during delivery or after birth, parenteral feeding, hospitalization, or psychological stress, for example. Microbiota dysbiosis may also result from gastrointestinal dysfunctions (digestive disorders, motility disorders, gastrointestinal reflux, slow gastrointestinal transit, oral feeding intolerance, constipation, diarrhea), Hirschsprung's disease, short bowel syndrome, gastrointestinal infection and inflammation affecting the gastrointestinal tract (such as necrotizing enterocolitis) and obstruction pathologies.

As well as being a consequence of gastrointestinal disorders, microbiota dysbiosis may actually cause them. Thus, microbiota dysbiosis may result in, for example, digestive disorders, motility disorders, gastrointestinal reflux, slow gastrointestinal transit, oral feeding intolerance, Hirschsprung's disease, and inflammation affecting the gastrointestinal tract (such as necrotizing enterocolitis) and obstruction pathologies.

The composition comprising at least one *Staphylococcus carnosus* strain for use according to the invention can prevent, or treatment of microbiota dysbiosis in a mammal at risk of or suffering from microbiota dysbiosis or prevent or treat a disorder associated with microbiota dysbiosis.

The disorders that may be treated or prevented by regulating microbiota dysbiosis include, for example, propensity to infection, allergy, type I diabetes mellitus, insulin resistance, type 2 diabetes, celiac disease, peripheral and central adiposity, obesity, necrotizing enterocolitis, inflammatory bowel disease, such as Crohn's disease and ulcerative colitis, and functional gastrointestinal disorders such as IBS, functional diarrhea, functional constipation, recurrent abdominal pain, and dyspepsia.

### EXAMPLES

The following non-limiting examples generally embodiments according to the present disclosure. In this regard, the inventors selected strains based on genome that could produce multiple tryptophan metabolites; those strains effectively produce those metabolites (in in vitro cultures); the metabolites that are produced are effective AHR receptor agonists (and to an effective balance); when testing in animal DSS model, the strains are indeed effective, and can not only reduce diseases score but only impact pro- and anti-inflammatory cytokines production.

### Example 1

The objective was *in silico* selection of bacterial strains possessing the necessary enzymes to produce tryptophan-derived molecules enabling the activation of the AHR and production of samples for metabolomic assessment. As detailed hereafter. Bacterial strains possessing the necessary genes encoding for enzymes producing tryptophan-derived metabolites, such as tryptamine, indole, indole-3-acetaldehyde and indole-3-acetic acid, were screened *in silico.* Strains belonging to the *Staphylococcus carnosus* species, a traditional meat starter culture, were identified as promising candidates, and samples were produced for further metabolomic assessment.

### Methods and Results

Publicly available protein references for enzymes catalyzing the formation of tryptamine, indole, indole-3-acetaldehyde, indole-3- acetic acid and 3-methyl-indole from tryptophan were retrieved from the publicly available Uniprot database (Table 1). No reference protein for the indole acetic acid oxidase was available in the public databases Uniprot, Swissprot, and BRENDA.

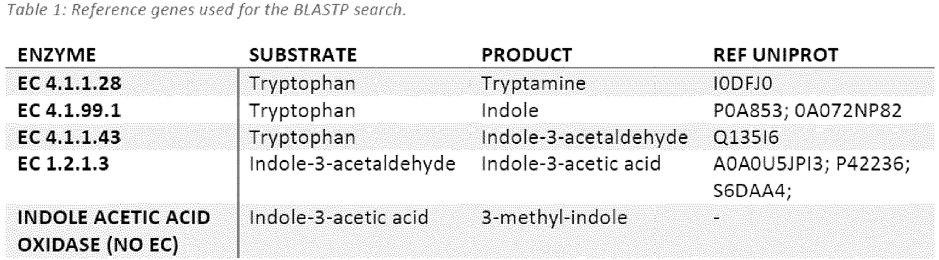

To identify genes, which could encode for above enzymes, a sequence similarity search was performed against all coding sequences of the genomes of the Nestlé Culture Collection (NCC). A total of 1,339 NCC strains from the showed hits against at least one of the enzymes. Forty-nine (49) strains showed hits against three of these enzymes.

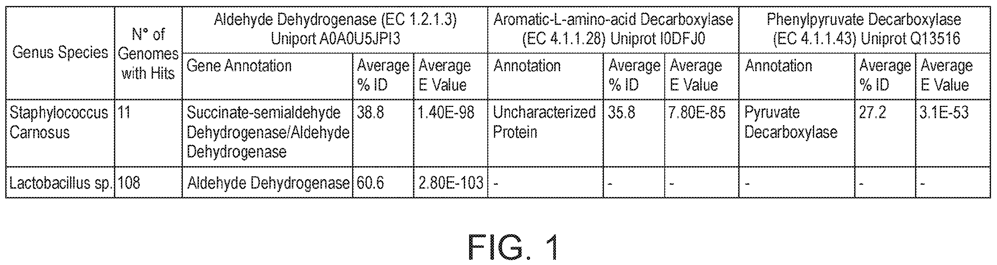

Some of the reference proteins used for the search were issued from organisms with a relatively low phylogenetical relationship to NCC strains, hence % of identity can be relatively low. However, annotation of those genes confirmed the correct potential activity of the enzymes encoded. A summary of the best NCC species is depicted in **FIG. 1****.**

As shown in **FIG. 1****,** *Staphylococcus carnosus* strains represent an interesting diversity with regard to their potential metabolite production and differ from probiotics discovered so far, mainly belonging to *Lactobacillus* species. *Staphylococcus carnosus* strains are of particular interest as they have been extensively used in the food industry as meat starters, and industrial compatible growth media was already previously developed.

Isolates of *S. carnosus* within the NCC and fully sequenced were selected for further testing. All these strains were reactivated under optimal conditions. Specifically, growth in Trypticase soy yeast (TSY) broth at 37°C for 10 hours to 48 hours at 220 rpm suited all strains. *Enterococcus feacalis* NCC 1978 was selected as negative control because it did not contain the necessary genes and it grew well in TSY.

Strains and their related supernatant were cultured in a homogeneous manner for further metabolomics assessment. Growth profile of each strain was therefore obtained in duplicates. Specifically, each strain was inoculated at 2% from a freshly grown culture and incubated at 37 °C with an agitation of 500 rpm.

For preparation of pellets and supernatants, each *S. carnosus* strain was cultured independently at 37 °C at 220 rpm, in 10 ml of TSY broth medium. Cells and supernatants were harvested three hours after their entry into the stationary phase, as determined earlier. Briefly, 10 ml of culture were centrifuged (3500 rpm, 20 min). Supernatant was collected and frozen at -80°C upon analysis.

To assess the specific quantities of tryptophan metabolites in various matrices, targeted LC-MS methods were developed for each metabolite and validated. In summary, NCC strains possessing the necessary genes encoding for enzymes producing tryptophan-derived metabolites, such as tryptamine, indole, indole-3-acetaldehyde and indole-3-acetic acid, were screened *in silico.* Strains, belonging to the *Staphylococcus carnosus* species, a traditional meat starter culture, were identified as promising candidates and samples were produced for further metabolomic assessment.

### Example 2

Probiotic strains of *Staphylococcus carnosus* are selected as the source of tryptophan metabolites to improve gut health in IBD patients and/ or the general population. The efficacy target is AHR activation and potential reduction of inflammation in the gut. The scientific hypothesis was that tryptophan metabolites produced by *S. carnosus* could result in the activation of Aryl Hydrocarbon Receptor (AHR) in gut epithelial cells to improve/enhance the integrity of gut epithelial cells.

The target population is either for inflammatory bowel disease (IBD) patients or the general population, with intended daily intake of *S. carnosus.*

### Methods and Results

The NCC genome database was screened for enzymes that catabolize tryptophan and related substrates. After *in vitro* growth and recovery studies, nine (9) strains were selected for further *in vivo* screening studies. The nine (9) strains selected were NCC836, NCC846, NCC888; NCC 971, NCC981, NCC1052, NCC1084, NCC1090, NCC1109 and NCC1978. After an animal study in a DSS model in mice, two strains (NCC971 and NCC1052) were chosen due to their high recovery rate in weight gain compared to other strains in the animal model. Strain NCC971 is also known as CNCM I-5398, strain NCC1053 is also known as CNCM I-5400, strain NCC846 is also known as CNCM I-5423, strain NCC982 is also known as CNCM I-5399, and strain NCC1090 is also known as CNCM I-5401.

*In silico* screenings for virulence factors and antibiotic resistance potential were internally assessed as below.

None of the genes for SE toxins - Staphylococcal enterotoxins (SEs), hemolysins, exfoliative toxin A (ETA), and toxic shock syndrome toxin 1 - were identified in the NCC 1052 and NCC971 strains (CNCM I-5400 and CNCM I-5398 respectively).

Whole genome sequences are available for all tested *S. carnosus* NCC strains. These additional *S. carnosus* NCC strains were screened *in silico* for the presence of the sequences of the five (5) above-mentioned enzymes found in NCC1052 and NCC971 strains (CNCM I-5400 and CNCM I-5398 respectively), using the same similarity search tool with same filtering thresholds. Most strains (16/18) were found positive for these five proteins.

Antibiotic resistance *in silico* screening for the 19 whole genome sequenced S. *carnosus* NCC strains including NCC1052 and NCC971 (CNCM I-5400 and CNCM I-5398 respectively) was performed using a reference database. Screening was done using "strict" and "loose" predictions. Since the confidence with "loose" predictions is lower, those hits were further filtered keeping all with %identity>70%. Most of the strains including NCC1052 and NCC971 have no hit with "strict" prediction. Using the "loose" predictions, hits were found with less than ten CARD database proteins linked with potential antibiotic resistance. For strains of NCC1052 and NCC971, *in silico* prediction did not identify any antibiotic resistance with 'strict' prediction.

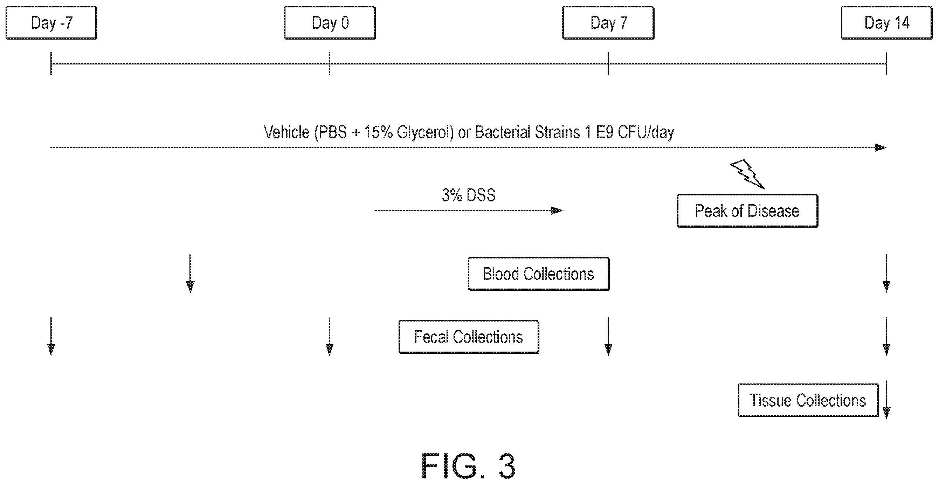

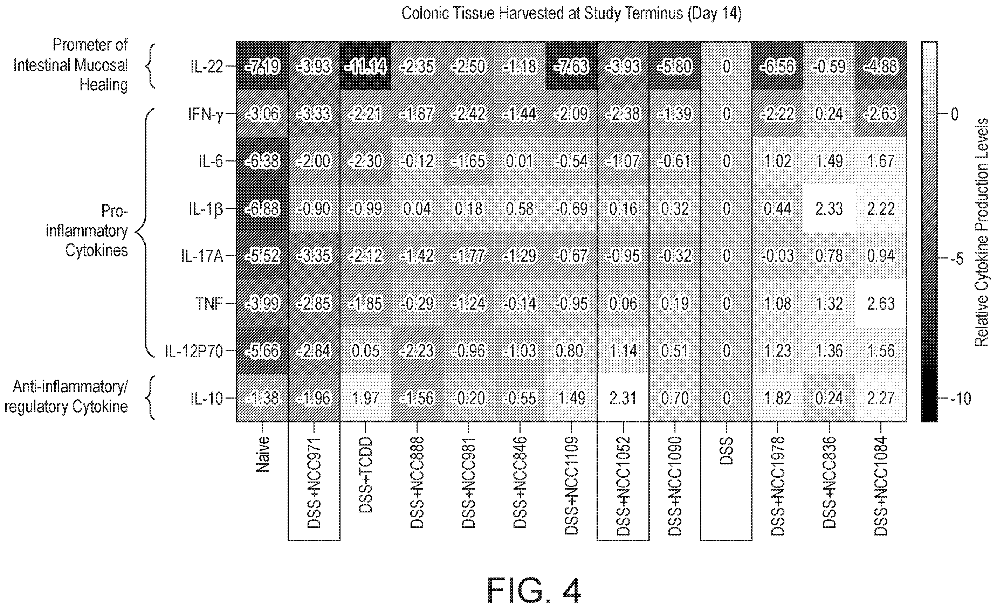

In order to screen the best candidates from nine (9) *S*. *carnosus* strains for improving gut health, a mouse model of IBD (Inflammatory Bowel Disease) with DSS (Dextran Sodium Sulphate) was employed **(****FIG. 3****).** Mice were pretreated with 10⁹ CFU of each strain daily for 7 days (D-7) via gavage, followed by treatment of DSS (3%) for 5 days along with the strains, then each strain only for another 9 days (D14). Readouts were daily weight change, daily composite Disease Activity Index (DAI), endoscopy and colon weight:length ratio score **(****FIG. 4****).** DAI is a composite score of weight loss, diarrhea, blood in stool, and activity level.

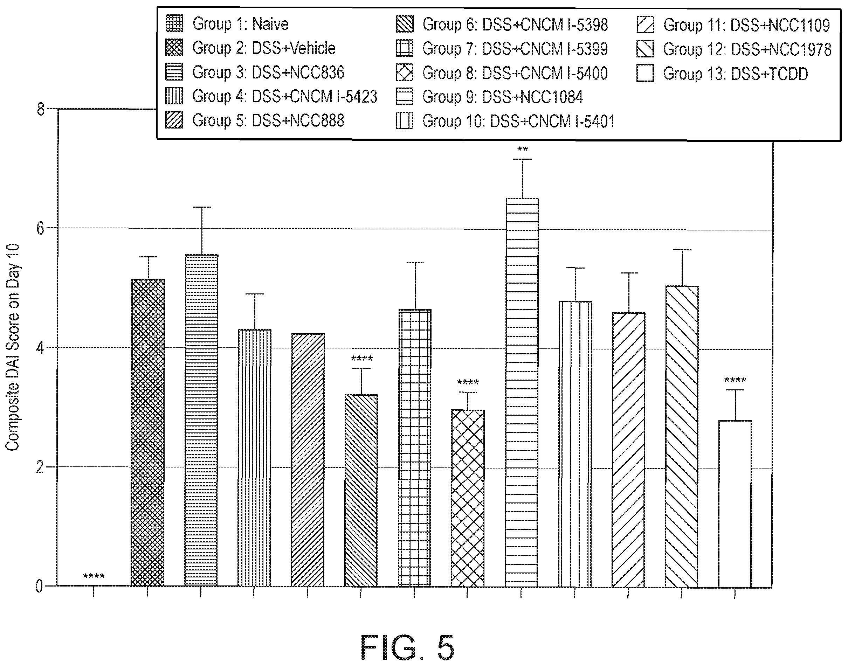

Composite DAI scores of NCC971 and NCC1052 (CNCM I-5398 and CNCM I-5400, respectively) were significantly lower than those of DSS control group at Day 10 **(****FIG. 5****).** No overt specific adverse effect or death was reported in this study.

### Example 3

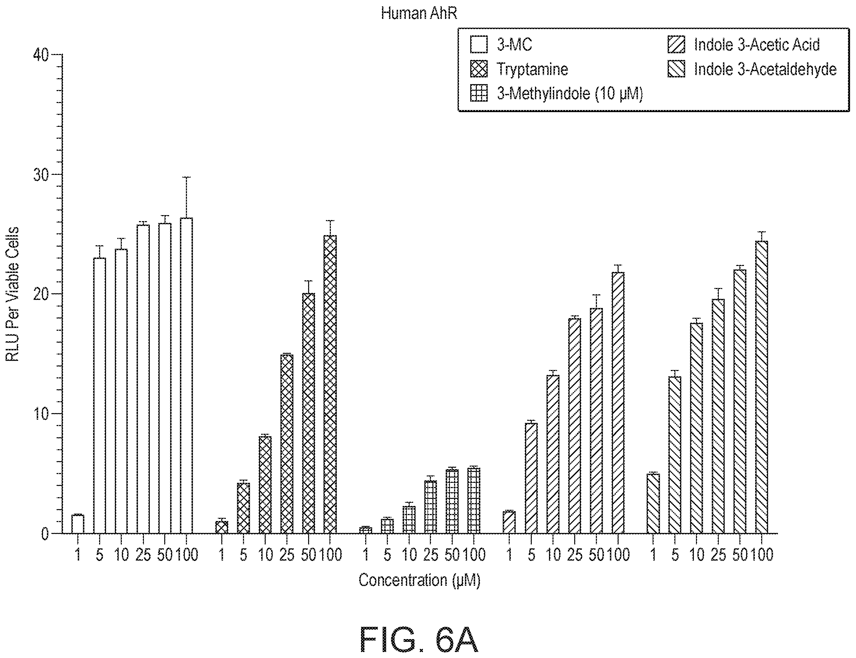

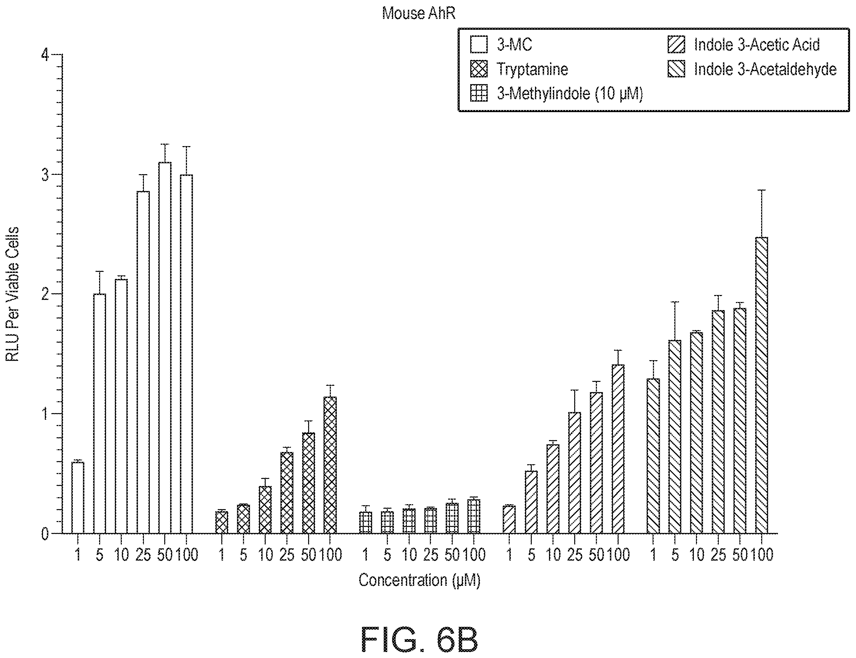

An *in vitro* AHR binding assay with a reporter gene readout was employed for characterization of the relative binding activities of tryptophan derived metabolites shown to be produced by *S. carnosus* and were compared to known toxic ligands. Both human and mouse AHR assays were utilized. Dioxin (TCDD) and Indirubin were used as reference compounds with strong binding and activation profiles. Commercially available metabolites were analyzed in dose response studies. Bacterial-derived tryptophan metabolites activate AHR in a dose-dependent manner **(****FIGS. 6A** **and** **6B****).**

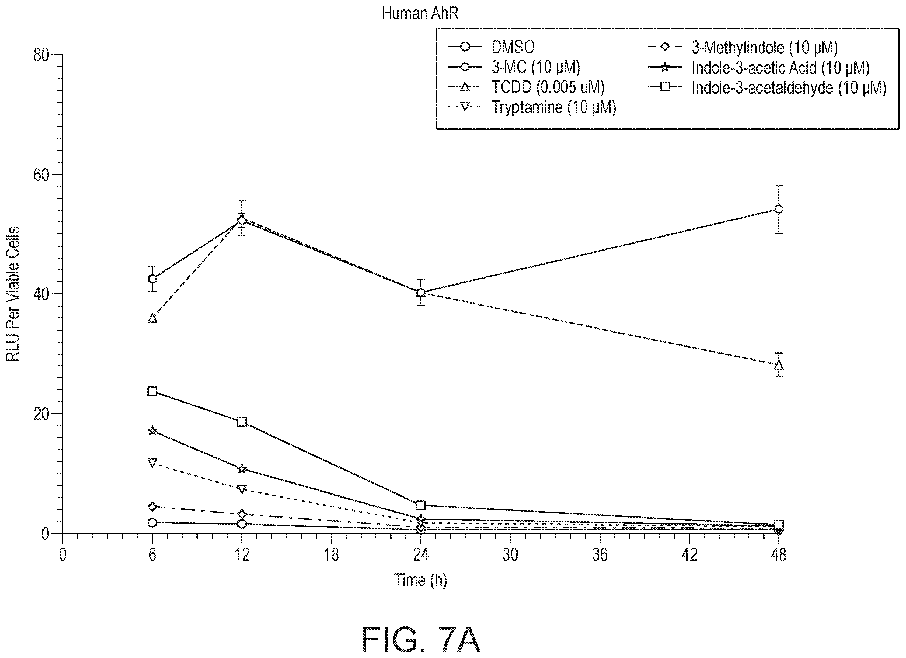

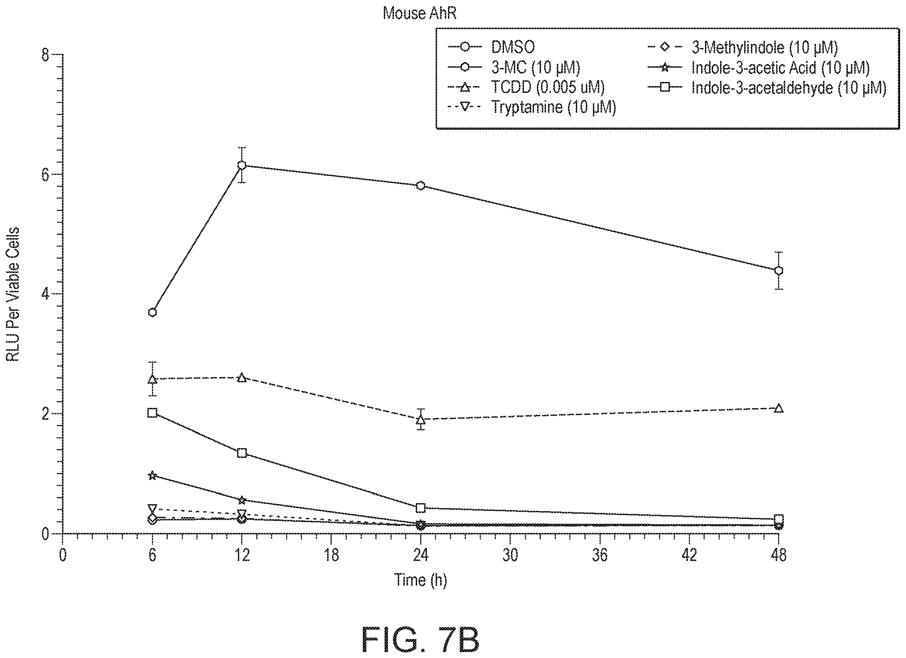

As shown in **FIGS. 7A** and **7B****,** tryptophan-derived metabolites, shown to be produced by *S. carnosus,* demonstrate low-level activation of AHR and with limited potentiation compared to TDD ( ≥ 2000X less active than TCDD (Dioxin))

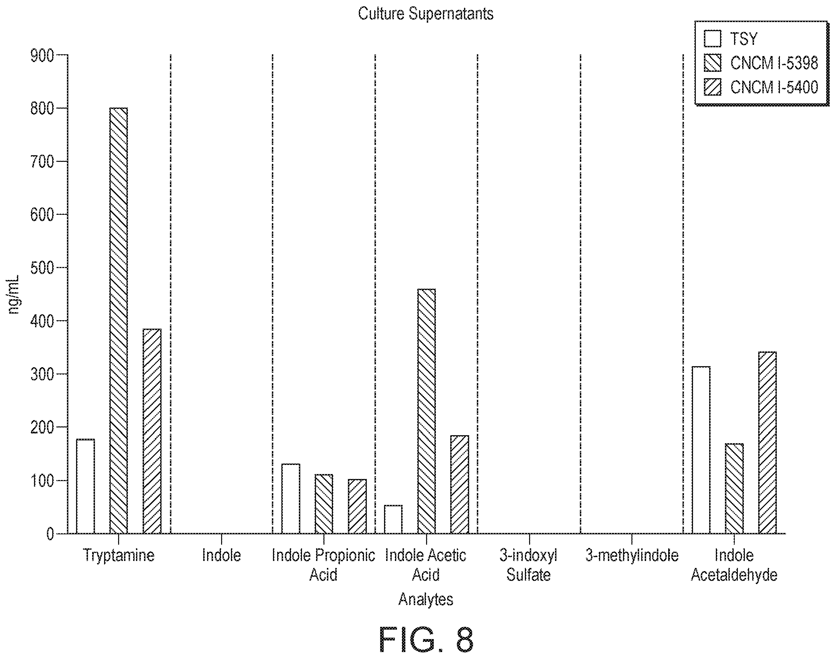

**FIG. 8** is a chart of results from an initial test of metabolites in culture supernatants.

### Example 4

This example relates to histopathology of mouse proximal and distal colon samples in a dextran sulfate sodium (DSS)-induced model of colitis, and to assess efficacy of treatment with NCC971 or NCC1052 (CNCM I-5400 and CNCM I-5398 respectively - alone or in combination) in the reduction of lesion severity; vehicle treatment was used as a negative control and anti-p40 treatment was used a positive control for test article comparison.

### Materials and Methods

Mice were administered 3% DSS on Days 0-5 and sacrifice was performed on Day 19. Treatments were given according to the experimental design below; animal numbers in parentheses indicate the number of animals submitted for histopathology evaluation if different from the number of animals in the study.

### Experimental Design

**Table 2**

| **Group** | 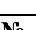 **Animals** | **Disease** | **Treatment** | **Dose** | **Schedule** | **Route** |
|---|---|---|---|---|---|---|
| 1 | 6 | No DSS | -- | -- | -- | -- |
| 2 | 20 | 3% DSS Days 0 to 5 | Vehicle | -- | QD Days -7 to 19 | PO |
| 3 | 20 | | NCC971 | 1x10⁹ CFU/strain 0.15 mL/mouse | | |
| 4 | 20 (19) | | NCC1052 | | | |
| 5 | 20 | | NCC971 + NCC1052 | | | |
| 6 | 12 (11) | | Anti-p40 | 10 mg/kg 0.1 mL/20 g | Q3D Days 6 to 19 | IP |

### Histology methods

Following sacrifice, colon samples were collected by the sponsor or designee according to the following protocol: the entire colon was rinsed and the most distal 5 cm portion was collected; of this 5 cm portion, the most proximal 2 cm and most distal 2 cm segments were isolated and fixed in 10% neutral buffered formalin. Colon segments from 96 mice were submitted to Inotiv Boulder. The most proximal and most distal segments of collected colon were trimmed into three cross sections per segment and both proximal and distal segments were embedded in the same block. Blocks were sectioned at approximately 5 µm and stained with hematoxylin and eosin (H&E) and periodic acid-Schiff (PAS).

### Pathology Methods

H&E-PAS-stained glass slides were evaluated by a board-certified veterinary pathologist using light microscopy. Colitis lesions (inflammation, gland necrosis/loss, erosion, hyperplasia, and edema) were given a severity score 0-5 (0=not present/normal, 1=minimal, 2=mild, 3=moderate, 4=marked, 5=severe). Individual histopathology scores were added together to determine a sum colitis score for each sample (range 0-25).

H&E-PAS slides were also scored for goblet cell abundance. This feature was scored in the remaining colonic glands (i.e. areas without colonic glands were excluded; that feature was assessed with gland loss, see above). The abundance of goblet cells was scored in comparison to the naive samples (score 0); increased goblet cells were scored in the positive range and decreased goblet cells were scored in the negative range; a sum score was also provided (decreased goblet cell score + increased goblet cell score).
- 3 = Diffuse loss of goblet cells
- 2 = Multifocal loss of goblet cells
- 1 = Focal loss of goblet cells
   0 = Goblet cell abundance approximately that of the naive control samples
   1 = Focal increase in goblet cells
   2 = Multifocal increase in goblet cells
   3 = Diffuse increase in goblet cells

Sub-cryptal mucosal measurements were performed in 3 representative areas per cross section (3 cross sections per proximal and distal segment = 9 measurements per colon segment; 6 total cross sections per animal = 18 measurements per animal). Mucosal measurements (µm) were collected by measuring the distance between the basement membrane of the mucosal crypts and the inner margin of the muscularis mucosae (sub-cryptal space). Areas that completely lacked epithelium (areas of gland loss/erosion) were not measured. Areas where the glandular epithelium had been replaced by stratified squamous epithelium (squamous metaplasia) were avoided, if possible. In cross sections where most or all of the epithelium was replaced by squamous epithelium, measurements were collected between the basement membrane of the squamous basal layer and the inner margin of the muscularis mucosae.

### Statistical Analysis

Data are presented as means ± standard error of the mean (SEM). Semi-quantitative severity scores were analyzed by a non-parametric Kruskal-Wallis test with pairwise Mann-Whitney exact *tests.* Two-tailed tests were utilized and significance was set at p≤0.05 for all tests.

### Results and Discussion

### Morphological findings

Administration of dextran sulfate sodium (DSS) to mice produced expected histologic lesions. These included subacute inflammation of the mucosa/submucosa, mucosal necrosis/gland loss, erosion, submucosal edema, and epithelial hyperplasia. Subacute inflammation was characterized by infiltration and aggregation of neutrophils, lymphocytes, and macrophages. Mucosal necrosis was characterized by damage to, necrosis of, or complete loss of colonic glands. **Erosions** were characterized by necrosis or loss of surface epithelium superficial to the muscularis mucosae. Submucosal edema was characterized by expansion of the submucosa by clear space or pale eosinophilic fluid, variably accompanied by dilation of lymphatic vessels and similar edematous expansion of the lamina propria. Epithelial hyperplasia was characterized by elongation of colonic glands, crypt and gland branching/arborization, epithelial cell basophilia, and increased numbers of epithelial mitotic figures. Some distal colon samples also exhibited squamous metaplasia, characterized by replacement of the glandular epithelium with stratified squamous epithelium.

Both increased and decreased numbers of goblet cells were seen in this study. In general, decreased numbers of goblet cells were seen in glands directly adjacent to or within regions of gland loss and erosion, whereas increased numbers of goblet cells were associated with some regions of hyperplasia.

Many of the "proximal" segments submitted for histopathologic evaluation included regions of either proximal colon (segments of colon with mucosal folds) and/or middle colon (lacking mucosal folds). Therefore, results pertaining to proximal segments of colon represent some combination of proximal and middle colon (see Table 1 for P:M ratio).

### Results

In both proximal colon and distal colon segments, lesions of colitis were absent in animals not receiving DSS (Group 1).

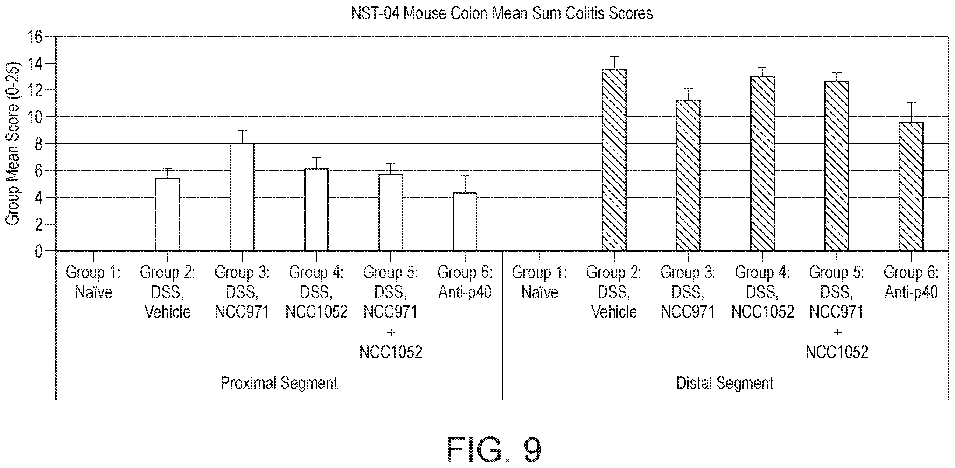

Sum scores **(****FIG. 9****)** were generally higher in distal colon segments than proximal colon segments. In comparison to the vehicle control (Group 2), statistically significant reductions in distal sum colitis scores were observed in mice treated with NCC971 (Group 3; p-value=0.050) or anti-p40 (Group 6; p-value=0.033). These reductions were not observed in the proximal colon (p-value>0.2; see Discussion). No statistically significant reductions were associated with NCC1052 (Group 4) treatment or the combination of NCC971 + NCC1052 (Group 5) treatment in the proximal segment (p-value>0.5) or distal segment (p-value>0.3) of colon.

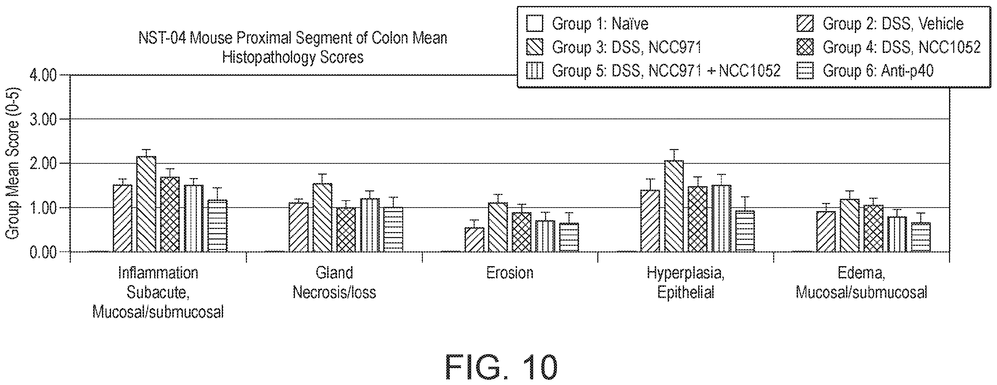

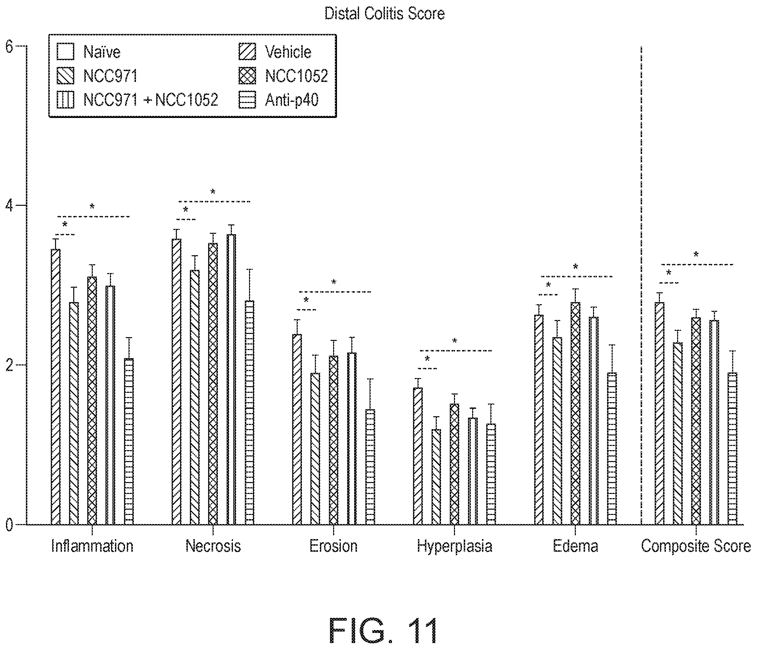

Similar amongst-group trends were observed in the component histopathology scores for the proximal segment **(****FIG. 10****)** and distal segment **(****FIG. 11****)** of colon.

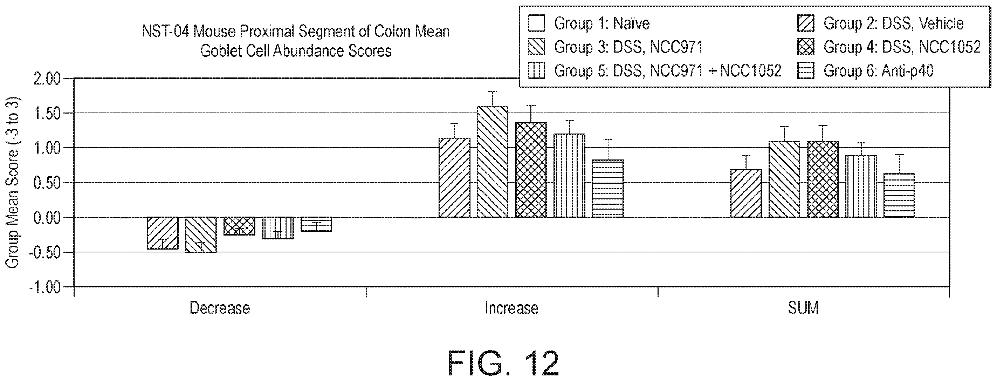

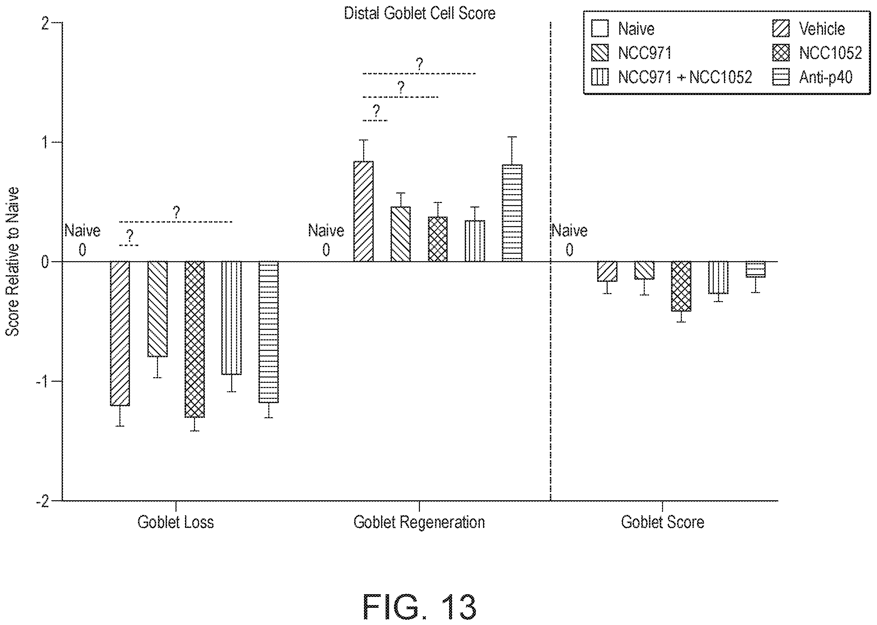

DSS-associated goblet cell abundance was primarily increased in the proximal colon **(****FIG. 12****)** and decreased in the distal colon **(****FIG. 13****).** No statistically significant differences were observed between the vehicle control (Group 2) and treatment groups (Groups 3-6) for sum abundance scores in either segment of colon (p-value>0.15).

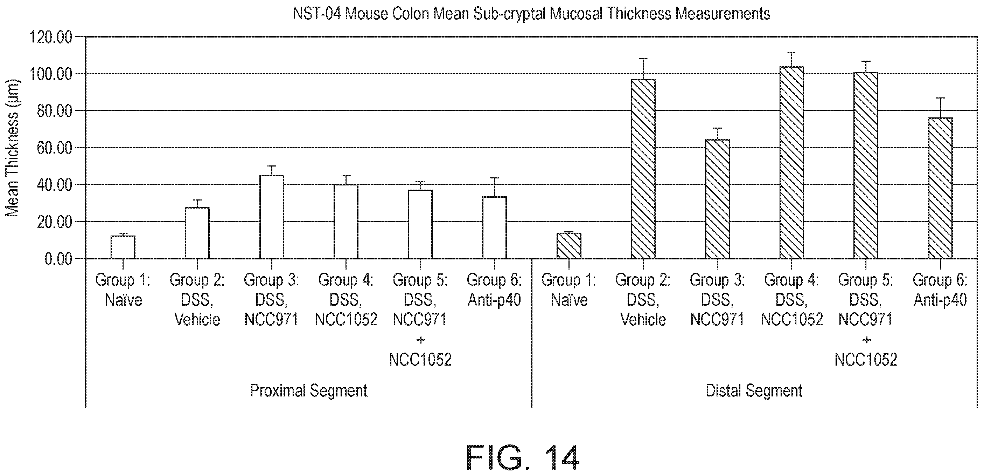

Sub-cryptal mucosal measurements **(****FIG. 14****)** were significantly larger in DSS-administered mice (Groups 2-6) compared to naïve mice (Group 1) in both the proximal (p-value=0.009) and distal segments (p-value<0.001). Amongst-group trends generally mirrored those seen with the sum colitis scores **(****FIG. 9****).** In comparison to the vehicle control (Group 2), statistically significant reductions in distal thickness measurements were observed in mice treated with NCC971 (Group 3; p-value=0.011). These reductions were not observed in the proximal colon (p-value>0.2). Treatment with the control compound, anti-p40 (Group 6), also yielded reduced sub-cryptal thickness measurements, but differences were not statistically significant (p-value=0.414). No reductions were associated with NCC1052 (Group 4) treatment or the combination of NCC971 + NCC1052 (Group 5) treatment in the proximal segment or distal segment of colon.

### Discussion

DSS administration in mice is known to cause greater disease severity the more distal the region of colon (i.e. colitis severity: proximal < middle < distal colon).₃ In this study, the proximal segment of colon was composed of varying ratios of true proximal and middle colon. The ratio of proximal to middle colon sections (P:M ratio) could have an effect on the overall severity scores observed for the proximal segment. Typically, the higher the P:M ratio (more proximal than middle colon), the lower the overall severity scores.

### Conclusions

DSS administration effectively induced expected histologic lesions. Test article results varied between the proximal and distal colon segments. NCC971 and anti-p40 treatment exhibited statistically significant reductions in colitis severity in the distal colon, but not the proximal colon. Treatment with NCC1052 or the combination of NCC971 + NCC1052 did not generally alter colitis severity in comparison to the vehicle control in either colon segment.

## Claims

1. At least one *Staphylococcus carnosus* strain for use in
(a) treating, preventing, reducing an incidence of, and/or reducing a severity of inflammation in an individual,
(b) preventing, treating or regulating microbiota dysbiosis in an individual at risk of or suffering from the microbiota dysbiosis;
(c) promoting intestinal mucosa healing; or
(d) preventing or treating a disorder associated with microbiota dysbiosis in an individual at risk of or having the disorder;
wherein the at least one *Staphylococcus carnosus* strain produces one or more of tryptamine, indole, indole propionic acid, 3-methylindole, indole-3-acetic acid or indole-3-acetaldehyde; and
wherein the at least one *Staphylococcus carnosus* strain comprises at least one of *S. carnosus* CNCM I-5398 or *S. carnosus* CNCM I-5400.

2. The strain for the use according to claim 1, wherein the at least one *Staphylococcus carnosus* strain is administered in a composition further comprising one or more additional probiotics, and the at least one *Staphylococcus carnosus* is a majority of a total amount of probiotics in the composition.

3. The strain for the use according to claim 1, wherein the at least one *Staphylococcus carnosus* strain is administered in a composition in which the at least one *Staphylococcus carnosus* is the only probiotic.

4. The strain for the use according to any one of claims 1 to 3, wherein the at least one *Staphylococcus carnosus* strain is administered to the individual by at least one route selected from the group consisting of oral, topical, enteral and parenteral.

5. The strain for the use according to any one of claims 1 and 2 to 4, wherein the at least one *Staphylococcus carnosus* strain is administered in a composition selected from the group consisting of a nutritionally complete product, a drink, a dietary supplement, a meal replacement, a food additive, a supplement to a food product, a powder for dissolution, an enteral nutrition product, an infant formula, and combinations thereof.

6. The strain for the use according to any one of claims 1 and 2 to 5, wherein the strain is for use in treating, preventing, reducing an incidence of, and/or reducing a severity of inflammation in an individual, and, wherein the inflammation is:
(a) selected from the group consisting of acute inflammation, skin inflammation, inflammatory bowel disease (IBD) including crohn's disease and/or ulcerative colitis, irritable bowel syndrome, liver inflammation, allergy, atopy, bone inflammation, rheumatoid arthritis, systemic lupus, Gougerot-Sjogren's syndrome, Reiter's syndrome, poliomyelitis, dermato-myositis, thyroiditis, Basedow, Hashimoto, type I diabetes, type II diabetes, Addison's disease, auto-immunes hepatitis, celiac disease, Biermer's disease, multiple sclerosis, myasthenia, eye inflammation, obesity-associated inflammation, age-related low-grade inflammation, Blau's syndrome, Alzheimer's disease, cardiovascular diseases, atherosclerosis, metabolic syndrome, gingivitis, paronditis, and combinations thereof;
(b) IBD; or
(c) IBD, wherein the IBD is Crohn's disease or ulcerative colitis.

7. The strain for the use according to any one of claims 1 and 2 to 5, wherein the strain is for use in preventing or treating a disorder associated with microbiota dysbiosis in an individual at risk of or having the disorder and wherein the disorder is selected from the group consisting of propensity to infection, allergy, type I diabetes mellitus, insulin resistance, type 2 diabetes, celiac disease, peripheral and central adiposity, obesity, necrotizing enterocolitis, inflammatory bowel disease, functional gastrointestinal disorders, and combinations thereof.

8. The strain for the use according to any one of claims 1 and 2 to 7, wherein the individual is selected from the group consisting of an infant, a child, an adolescent, an adult and an elderly person.

9. The strain for the use according to any one of claims 1 and 2 to 8, wherein the at least one *Staphylococcus carnosus* strain is administered in a composition further comprising at least one component selected from the group consisting of a prebiotic, an amino acid, a protein, a nucleotide, a fish oil, a non-marine source of omega-3 fatty acids, a phytonutrient, an antioxidant, and mixtures thereof.

## Patentansprüche

1. Mindestens ein *Staphylococcus carnosus*-Stamm zur Verwendung bei
(a) einem Behandeln, Vorbeugen, Verringern einer Inzidenz von und/oder Verringern einer Schwere einer Entzündung in einem Individuum,
(b) dem Vorbeugen, dem Behandeln oder einem Regulieren einer Mikrobiota-Dysbiose in einem Individuum, das ein Risiko für die Mikrobiota-Dysbiose aufweist oder daran leidet;
(c) einem Fördern eines Heilens einer Darmschleimhaut; oder
(d) dem Vorbeugen oder Behandeln einer Störung, die mit Mikrobiota-Dysbiose verknüpft ist, in einem Individuum, das ein Risiko für die Störung aufweist oder diese aufweist;
wobei der mindestens eine *Staphylococcus carnosus*-Stamm eines oder mehrere von Tryptamin, Indol, Indolpropionsäure, 3-Methylindol, Indol-3-essigsäure oder Indol-3-acetaldehyd produziert; und
wobei der mindestens eine *Staphylococcus carnosus*-Stamm mindestens einen von *S. carnosus* CNCM I-5398 oder *S. carnosus* CNCM I-5400 umfasst.

2. Stamm für die Verwendung nach Anspruch 1, wobei der mindestens eine *Staphylococcus carnosus*-Stamm in einer Zusammensetzung verabreicht wird, ferner umfassend ein oder mehrere zusätzliche Probiotika, und der mindestens eine *Staphylococcus carnosus* eine Mehrheit einer Gesamtmenge von Probiotika in der Zusammensetzung ist.

3. Stamm für die Verwendung nach Anspruch 1, wobei der mindestens eine *Staphylococcus carnosus*-Stamm in einer Zusammensetzung verabreicht wird, in der der mindestens eine *Staphylococcus carnosus* das einzige Probiotikum ist.

4. Stamm für die Verwendung nach einem der Ansprüche 1 bis 3, wobei der mindestens eine *Staphylococcus carnosus*-Stamm dem Individuum durch mindestens einen Weg verabreicht wird, der aus der Gruppe ausgewählt ist, bestehend aus oral, topisch, enteral und parenteral.

5. Stamm für die Verwendung nach einem der Ansprüche 1 und 2 bis 4, wobei der mindestens eine *Staphylococcus carnosus*-Stamm in einer Zusammensetzung verabreicht wird, die aus der Gruppe ausgewählt ist, bestehend aus einem ernährungsphysiologisch vollständigen Produkt, einem Getränk, einem Nahrungsergänzungsmittel, einem Mahlzeitenersatz, einem Lebensmittelzusatzstoff, einem Ergänzungsmittel zu einem Lebensmittelprodukt, einem Pulver für eine Auflösung, einem enteralen Ernährungsprodukt, einer Säuglingsanfangsnahrung und Kombinationen davon.

6. Stamm für die Verwendung nach einem der Ansprüche 1 und 2 bis 5, wobei der Stamm zur Verwendung bei dem Behandeln, Vorbeugen, Verringern einer Inzidenz von und/oder dem Verringern einer Schwere der Entzündung in einem Individuum ist und wobei die Entzündung ist:
(a) aus der Gruppe ausgewählt, bestehend aus akuter Entzündung, Hautentzündung, entzündlicher Darmerkrankung (IBD), die Morbus Crohn und/oder Colitis ulcerosa einschließt, Reizdarmsyndrom, Leberentzündung, Allergie, Atopie, Knochenentzündung, rheumatoider Arthritis, systemischem Lupus, Gougerot-Sjögren-Syndrom, Reiter-Syndrom, Poliomyelitis, Dermatomyositis, Thyreoiditis, Basedow, Hashimoto, Typ-I-Diabetes, Typ-II-Diabetes, Addison-Krankheit, Autoimmunhepatitis, Zöliakie, Biermer-Krankheit, multipler Sklerose, Myasthenie, Augenentzündung, mit Adipositas verknüpfter Entzündung, altersbedingter niedriggradiger Entzündung, Blau-Syndrom, Alzheimer-Krankheit, kardiovaskulären Erkrankungen, Atherosklerose, metabolischem Syndrom, Gingivitis, Parodontitis und Kombinationen davon;
(b) IBD; oder
(c) IBD, wobei die IBD Morbus Crohn oder Colitis ulcerosa ist.

7. Stamm für die Verwendung nach einem der Ansprüche 1 und 2 bis 5, wobei der Stamm zur Verwendung bei dem Vorbeugen oder Behandeln einer Störung, die mit Mikrobiota-Dysbiose verknüpft ist, in einem Individuum ist, das ein Risiko für die Störung aufweist oder diese aufweist, und wobei die Störung aus der Gruppe ausgewählt ist, bestehend aus Infektionsneigung, Allergie, Typ-I-Diabetes mellitus, Insulinresistenz, Typ-2-Diabetes, Zöliakie, peripherer und zentraler Adipositas, Adipositas, Enterocolitis necroticans, entzündlicher Darmerkrankung, funktionellen gastrointestinalen Störungen und Kombinationen davon.

8. Stamm für die Verwendung nach einem der Ansprüche 1 und 2 bis 7, wobei das Individuum aus der Gruppe ausgewählt ist, bestehend aus einem Säugling, einem Kind, einem Jugendlichen, einem Erwachsenen und einer älteren Person.

9. Stamm für die Verwendung nach einem der Ansprüche 1 und 2 bis 8, wobei der mindestens eine *Staphylococcus carnosus*-Stamm in einer Zusammensetzung verabreicht wird, ferner umfassend mindestens eine Komponente, die aus der Gruppe ausgewählt ist, bestehend aus einem Präbiotikum, einer Aminosäure, einem Protein, einem Nukleotid, einem Fischöl, einer nicht marinen Quelle von Omega-3-Fettsäuren, einem Phytonährstoff, einem Antioxidans und Mischungen davon.

## Revendications

1. Au moins une souche de *Staphylococcus carnosus* pour une utilisation dans
(a) le traitement, la prévention, la réduction de l'incidence et/ou la réduction de la sévérité d'une inflammation chez un individu,
b) la prévention, le traitement ou la régulation d'une dysbiose du microbiote chez un individu à risque ou souffrant de la dysbiose du microbiote ;
c) la promotion de la cicatrisation de la muqueuse intestinale ; ou
d) la prévention ou le traitement d'un trouble associé à une dysbiose du microbiote chez un individu à risque ou atteint de ce trouble ;
dans laquelle l'au moins une souche de *Staphylococcus carnosus* produit un ou plusieurs parmi tryptamine, acide indole-propionique, 3-méthylindole, acide indole-3-acétique ou indole-3-acétaldéhyde ; et
dans laquelle l'au moins une souche de *Staphylococcus carnosus* comprend au moins l'une parmi *S. carnosus* CNCM I-5398 ou *S. carnosus* CNCM I-5400.

2. Souche pour l'utilisation selon la revendication 1, dans laquelle l'au moins une souche de *Staphylococcus carnosus* est administrée dans une composition comprenant en outre un ou plusieurs probiotiques supplémentaires, et l'au moins un *Staphylococcus carnosus* constitue une majorité d'une quantité totale de probiotiques dans la composition.

3. Souche pour l'utilisation selon la revendication 1, dans laquelle l'au moins une souche de *Staphylococcus carnosus* est administrée dans une composition dans laquelle l'au moins une souche de *Staphylococcus carnosus* est le seul probiotique.

4. Souche pour l'utilisation selon l'une quelconque des revendications 1 à 3, dans laquelle l'au moins une souche de *Staphylococcus carnosus* est administrée à l'individu par au moins une voie choisie dans le groupe constitué des voies orale, topique, entérale et parentérale.

5. Souche pour l'utilisation selon l'une quelconque des revendications 1 et 2 à 4, dans laquelle l'au moins une souche de *Staphylococcus carnosus* est administrée dans une composition choisie dans le groupe constitué d'un produit nutritionnellement complet, une boisson, un complément alimentaire, un substitut de repas, un additif alimentaire, un complément à un produit alimentaire, une poudre destinée à être dissoute, un produit de nutrition entérale, une préparation pour nourrissons, et des combinaisons de ceux-ci.

6. Souche pour l'utilisation selon l'une quelconque des revendications 1 et 2 à 5, dans laquelle la souche est pour destinée à une utilisation dans le traitement, la prévention, la réduction d'une incidence et/ou la réduction d'une sévérité d'une inflammation chez un individu, et, dans laquelle l'inflammation est :
a) choisie dans le groupe constitué d'inflammation aiguë, inflammation cutanée, maladie inflammatoire chronique de l'intestin (MICI), y compris la maladie de Crohn et/ou la colite ulcéreuse, syndrome du côlon irritable, inflammation du foie, allergie, atopie, inflammation osseuse, polyarthrite rhumatoïde, lupus disséminé, syndrome de Gougerot-Sjögren, syndrome de Reiter, poliomyélite, dermatomyosite, thyroïdite, maladie de Basedow, thyroïdite de Hashimoto, diabète de type I, diabète de type II, maladie d'Addison, hépatite auto-immune, maladie cœliaque, maladie de Biermer, sclérose en plaques, myasthénie, inflammation oculaire, inflammation associée à l'obésité, inflammation de bas grade liée à l'âge, syndrome de Blau, maladie d'Alzheimer, maladies cardiovasculaires, athérosclérose, syndrome métabolique, gingivite, parodontite, et combinaisons de ceux-ci ;
b) une MICI ; ou
(c) une MICI, dans laquelle la MICI est la maladie de Crohn ou la colite ulcéreuse.

7. Souche pour l'utilisation selon l'une quelconque des revendications 1 et 2 à 5, dans laquelle la souche est destinée à une utilisation dans la prévention ou le traitement d'un trouble associé à une dysbiose du microbiote chez un individu à risque ou présentant le trouble et dans laquelle le trouble est choisi dans le groupe constitué de propension à l'infection, allergie, diabète sucré de type I, insulinorésistance, diabète de type 2, maladie cœliaque, adiposité périphérique et centrale, obésité, entérocolite nécrosante, maladie inflammatoire chronique de l'intestin, troubles gastro-intestinaux fonctionnels et combinaisons de ceux-ci.

8. Souche pour l'utilisation selon l'une quelconque des revendications 1 et 2 à 7, dans laquelle l'individu est choisi dans le groupe constitué d'un nourrisson, d'un enfant, d'un adolescent, d'un adulte et d'une personne âgée.

9. Souche pour l'utilisation selon l'une quelconque des revendications 1 et 2 à 8, dans laquelle l'au moins une souche de *Staphylococcus carnosus* est administrée dans une composition comprenant en outre au moins un composant choisi dans le groupe constitué d'un prébiotique, d'un acide aminé, d'une protéine, d'un nucléotide, d'une huile de poisson, d'une source non marine d'acides gras oméga-3, d'un phytonutriment, d'un antioxydant, et de mélanges de ceux-ci.
